# EUROPEAN PATENT APPLICATION

(11) **EP 2 281 904 A1**
(43) Date of publication of application: **09.02.2011**
(21) Application number: 09166590.1
(22) Date of filing: 28.07.2009
(51) Int. Cl.: C12Q 1/68

(54) **In vitro method for predicting whether a compound is genotoxic in vivo.**

(71) Applicant: Universiteit Maastricht, 6211 LK Maastricht (NL)
(72) Inventor: Kleinjans, Joseph Catharina Stephanus, 6221 AR Maastricht (NL); Van Delft, Joseph Henri Marie, 6221 GJ Maastricht (NL); Mathijs, Karen, 3990 Hamont (BE)
(74) Representative: Habets, Winand

(57) **Abstract**

The invention is in the field of genomics and it provides an in vitro method for predicting whether a compound is genotoxic in vivo. It provides a method that employs the analysis of expression profiles of primary mouse hepatocytes as an in vitro system to discriminate false GTX compounds from true GTX carcinogens. It was found that differential expression of a number of genes could reliably predict whether a compound was a true genotoxic compound.

## Description

### Field of the invention

The invention is in the field of genomics and it provides an in vitro method for predicting whether a compound is genotoxic (GTX) in vivo

### Background of the invention

The classic 2 year rodent bioassay is the standard test for identifying the carcinogenic potential of chemical compounds. Such tests are time-consuming and costly. Moreover, they require the sacrifice of many animal lives. In vitro systems are therefore preferred; however, there is no reliable in vitro method for accurately predicting the genotoxicity of a compound in vivo.(1,2).

Well-established in vitro systems frequently used to identify the genotoxic potential of chemical compounds are for instance the bacterial Ames test, the mouse lymphoma assay, the micronucleus test and the chromosomal aberration test (3).

These classic in vitro genotoxicity tests, however, have been shown to generate an extremely high false positive rate when compared to in vivo carcinogenicity data. (3). False positive in this context means that the compound yields a positive result in the in vitro assay whereas it is negative for genotoxicity in an in vivo assay.

Because of the low predictive value of current in vitro assays, a compound that tested positive in an in vitro assay has to be retested in an in vivo assay in order to verify whether the compound is a true genotoxic (GTX) compound. This generates a lot of extra costs and efforts, as well as the sacrifice of many animal lives.

Therefore, new and more predictive in vitro systems are desired in the art which are capable of reliably discriminating genotoxins from non-genotoxins.

### Summary of the invention

The present invention employs the analysis of expression profiles of primary mouse hepatocytes as an in vitro system to discriminate false GTX compounds from true GTX carcinogens. It was found that differential expression of a number of genes could reliably predict whether a compound was a true genotoxic compound.

Hence, the invention relates to an in vitro method for predicting whether a compound is a true in vivo genotoxic compound comprising the steps of
a. providing primary mouse hepatocytes
b. providing an assay capable of determining the expression of at least one gene selected from the group consisting of the genes provided in table 1 and table 2,
c. contacting the primary mouse hepatocytes with a test compound
d. Isolating RNA from said mouse hepatocytes at two consecutive moments in time and
e. determining the expression of at least one gene selected from the group consisting of the genes provided in table 1 and table 2,
f. repeating steps a to e at least 2 times to obtain at least three measurements of expression of said at least one gene
g. comparing the at least 3 measurements of expression of said at least one gene with and without the test compound at the two consecutive moment in time with data obtained from known true genotoxic compounds by means of a supervised clustering method,
h. obtaining at least 6 preliminary predictions for genotoxicity of the compound i. predicting the true in vivo genotoxicity of the test compound according to the schedule of table 3.

### Detailed description of the invention

Chemical compounds, which are able to cause gene mutations or chromosomal damage in vivo are herein defined as true genotoxins (true GTX) (6, 12). False positive genotoxins (false positive GTX or false GTX) are herein defined as compounds that are not capable of causing gene mutations or chromosomal damage in vivo, but are positive in an in vitro assay for genotoxicity.

Gene mutations or chromosomal damage may occur when the compound covalently binds to DNA in vivo. Such binding to DNA may be not or incorrectly repaired which may lead to mutations accumulating in time and ultimately inducing the formation of tumors (6, 12).

The present invention employs the analysis of expression profiles of primary mouse hepatocytes as an in vitro system to discriminate false GTX compounds from true GTX carcinogens. It was found that differential expression of a number of genes could reliably predict whether a compound was a true genotoxic compound. So as a first step in the method according to the invention, a culture of primary mouse hepatocytes is provided. The skilled person is aware of the various methods that may be used to obtain a culture of primary mouse hepatocytes. The examples provided herein may provide additional guidance.

In a further step of the method according to the invention, an assay is provided capable of determining the expression of at least one gene selected from the group consisting of the genes provided in table 1 and table 2. Assays that may determine gene expression are also known in the art. Such an assay may consist of an assay capable of determining the expression of a single gene, such as a single PCR- based assay or a hybridization assay. In the alternative, a multiplex assay may be used, consisting of a plurality of different assays that can be performed simultaneously. This allows for the determination of simultaneous expression of more than one gene. Even more advantageously, the assay is a nucleic acid microarray such as a DNA microarray, such as a GeneChip® provided by Affymetrix.

The genes provided in table 1 and 2 are readily accessible and identifiable for a person skilled in the art by their trivial name only. For reason of convenience, also the Genebank accession codes and Entrez Gene ID are given in table 1 and table 2. Primary sequences of these genes are published and can easily be retrieved from numerous public sources, such as Genebank.

**TABLE 1 Genes suitable in the method according to the invention**

| **GENEBANK** **Access code** | **GENE SYMBOL** | **ENTREZ GENE ID** |
|---|---|---|
| AK010447 | Smyd3 | 69726 |
| BB318221 | Zdhhc14 | 224454 |
| BG261907 | Large | 16795 |
| Y15910 | Diap2 | 54004 |
| AV095209 | Mthfd1l | 270685 |
| AK019979 | 2610528E23Rik | 66497 |
| BC016073 | Cdkal1 | 68916 |
| BB821363 | Scfd2 | 212986 |
| Al596632 | Ptprg /// LOC632664 | 19270 |
| AW986246 | Maoa | 17161 |
| NM_028803 | Gbe1 | 74185 |
| AV141095 | 1110033MO5Rik | 68675 |
| AF000969 | Cadps2 | 320405 |
| BB526605 | Mipol1 | 73490 |
| NM_008576 | Abcc1 | 17250 |
| BG070887 | Gtdc1 | 227835 |
| AW543460 | Pard3 | 93742 |
| BC016265 | Ube2e2 | 218793 |
| AV223474 | Zdhhc14 | 224454 |
| Al987929 | Ndrg1 | 17988 |
| AK009736 | Gpr137b /// LOC664862 /// LOC673335 | |
| AK007766 | 1810044A24Rik | 76510 |
| AK004419 | Fbxl17 | 50758 |
| AV173571 | 1700106N22Rik | 73582 |
| BB308836 | Ppm1l | 242083 |
| BC004827 | Psat1 | 107272 |
| AW240761 | Tbc1d5 | 72238 |
| BG066903 | Kif16b | 16558 |
| NM_025770 | Atg10 | 66795 |
| BC025915 | Cova1 | 209224 |
| NM_018770 | Igsf4a | 54725 |
| AF022072 | Grb10 | 14783 |
| BC025837 | Sbk1 | 104175 |
| BG076151 | Ppm1d | 53892 |
| BF719766 | Thyn1 | 77862 |
| AV377066 | 9130221J18Rik | 102123 |
| BG065754 | Ccng1 | 12450 |
| BC025501 | Aaas | 223921 |
| NM_134188 | Acot2 | 171210 |
| NM_021451 | Pmaip1 | 58801 |
| BC026422 | Tgm1 | 21816 |
| BC015270 | Hist2h3c2 | 97114 |
| NM_053168 | Trim11 | 94091 |
| BB027848 | 4732466D17Rik | 212933 |
| AV327248 | Zfp365 /// LOC674611 | |
| AV219418 | Ldhb | 16832 |
| BG069873 | Gnb1l | 13972 |
| AF204959 | Cyp3a25 /// LOC622249 | 56388 |
| NM_030697 | Ankrd47 | 80880 |
| BM198879 | Ercc5 | 22592 |
| AW543723 | | |
| AK014608 | 4632434111Rik | 74041 |
| AV298304 | Homez | 239099 |
| BC012260 | Psmf1 | 228769 |
| NM_013866 | Zfp385 | 29813 |
| AF065917 | Lif | 16878 |
| AF297615 | Ggta1 | 14594 |
| BB770528 | Rai2 | 24004 |
| BC012247 | Dcxr | 67880 |
| NM_011316 | Saa4 | 20211 |
| NM_007987 | Fas | 14102 |
| B1660702 | Ell3 | 269344 |
| BM230508 | A030007D23Rik | 319530 |
| Al594683 | Dmn | 233335 |
| NM_011176 | St14 | 19143 |
| BB463610 | 4632434111Rik | 74041 |
| BC019882 | Acaa1b | 235674 |
| AK007854 | 1810053B23Rik | 69857 |
| BC010462 | BC010462 | 209588 |
| BB043558 | 9230114K14Rik | 414108 |
| NM_008522 | Ltf | 17002 |
| NM_012006 | Acot1 | 26897 |
| BB275142 | AW456874 | 218232 |
| BC008626 | Icam1 | 15894 |
| Bl651416 | Cdc42bpg | 240505 |
| AK005731 | 1700007K13Rik | 69327 |

**TABLE 2 Genes suitable in the method according to the invention**

| **GENEBANK** **Access code** | **GENE SYMBOL** | **ENTREZ GENE ID** |
|---|---|---|
| AK005731 | 1700007K13Rik | 69327 |
| Bl651416 | Cdc42bpg | 240505 |
| NM_008522 | Ltf | 17002 |
| BB043558 | 9230114K14Rik | 414108 |
| NM_007987 | Fas | 14102 |
| BC022148 | Ces5 | 234673 |
| BC019882 | Acaa1b | 235674 |
| BB463610 | 4632434l11Rik | 74041 |
| BM230508 | A030007D23Rik | 319530 |
| Al594683 | Dmn | 233335 |
| AV327248 | Zfp365 /// LOC674611 | |
| BE956581 | Cpt1c | 78070 |
| NM_011176 | St14 | 19143 |
| BM200015 | Hsdl2 | 72479 |
| BB223872 | Bscl2 | 14705 |
| AF297615 | Ggta1 | 14594 |
| BC027026 | Cdkn2c | 12580 |
| NM_012006 | Acot1 | 26897 |
| AK014608 | 4632434l11Rik | 74041 |
| BC012247 | Dcxr | 67880 |
| BC027121 | Spbc25 | 66442 |
| BG797099 | Ddit4l | 73284 |
| BB743970 | BC015286 | 234669 |
| BF719766 | Thyn1 | 77862 |
| BC027185 | 2210023G05Rik | 72361 |
| AF033112 | Siva | 30954 |
| BG065754 | Ccng1 | 12450 |
| BB781615 | 6530418L21Rik | 109050 |
| BC013893 | Masp2 | 17175 |
| BC003284 | Wdr21 | 73828 |
| BC006713 | Dgka | 13139 |
| NM_011075 | Abcb1b | 18669 |
| BB009155 | | |
| BG967046 | Tbc1d2 | 381605 |
| NM_030697 | Ankrd47 | 80880 |
| BB275142 | AW456874 | 218232 |
| AV246296 | Eda2r | 245527 |
| NM_013738 | Plek2 | 27260 |
| NM_018881 | Fmo2 | 55990 |
| BM936480 | Fmo2 | 55990 |
| BM198879 | Ercc5 | 22592 |
| AK018383 | Tmem19 | 67226 |
| AV254764 | | |
| BC021352 | Plod2 | 26432 |
| BB027848 | 4732466D17Rik | 212933 |
| AK017734 | Tmem14a | 75712 |
| AF069954 | Bscl2 | 14705 |
| BB770528 | Rai2 | 24004 |
| NM_009897 | Ckmt1 | 12716 |
| AK007854 | 1810053B23Rik | 69857 |
| Bl966443 | Itm2a | 16431 |
| NM_013929 | Siva | 30954 |
| BG076151 | Ppm1d | 53892 |
| AV251625 | Ddit4l | 73284 |
| AV219418 | Ldhb | 16832 |
| NM_011316 | Saa4 | 20211 |
| NM_007980 | Fabp2 | 14079 |
| BB046347 | Mycbp | 56309 |
| AF335325 | Ddit4l | 73284 |
| AK010738 | Ascl2 | 17173 |
| NM_134188 | Acot2 | 171210 |
| NM_008935 | Prom1 | 19126 |
| BB140436 | Slc16a10 | 72472 |
| NM_019738 | Nupr1 | 56312 |
| X62701 | Plaur | 18793 |
| AV141095 | 1110033M05Rik | 68675 |
| Al747296 | Gmds | 218138 |
| BC005552 | Asns | 27053 |
| BB458460 | Chchd6 | 66098 |
| BG076333 | Mthfd2 | 17768 |
| AK019979 | 2610528E23Rik | 66497 |
| AV095209 | Mthfd1l | 270685 |
| | Phgdh /// LOC668771 /// LOC671972 /// | |
| AV216768 | LOC673015 | |
| AV221299 | Gfra1 | 14585 |
| BQ174991 | Chsy1 | 269941 |
| NM_013642 | Dusp1 | 19252 |
| L21027 | Phgdh/// LOC666422 /// LOC666875 /// LOC669985 /// LOC671102 /// LOC673015 /// LOC675010 | 236539 |
| BB204486 | Phgdh /// LOC382931 /// LOC384524 /// LOC385344 /// LOC547171 /// LOC627427 /// LOC666422 /// LOC6 | |
| BC025169 | Chac1 | 69065 |
| BC026131 | Slc7a5 | 20539 |
| BC010318 | Pck2 | 74551 |
| BB730977 | Cachd1 | 320508 |
| AA561726 | Phgdh /// LOC668771 /// LOC670155 /// LOC671972 /// LOC673015 | |
| BC012955 | Trib3 | 228775 |
| BC004827 | Psat1 | 107272 |
| NM_007556 | Bmp6 | 12161 |
| NM_134147 | D930010J01Rik | 107227 |
| AV173869 | D14Ertd171e | 238988 |
| AF022072 | Grb10 | 14783 |
| BC019379 | Gprk5 | 14773 |
| AK010447 | Smyd3 | 69726 |
| BC017615 | Slc24a3 | 94249 |
| BB246912 | 1700112E06Rik | 76633 |
| AF000969 | Cadps2 | 320405 |
| BG066491 | Fhod3 | 225288 |
| AF055573 | Fhit | 14198 |
| NM_053122 | Immp2l | 93757 |

In another step of the method according to the invention, the compound to be tested is contacted with the primary mouse hepatocytes. The skilled person will be aware of the metes and bounds of this step. In the examples section, the concentrations used for 10 true and false GTX compounds are provided as guidance. in general, the use of cytotoxic concentrations should be avoided. The skilled person will know how to avoid using cytotoxic concentrations of test compounds.

It was found to be useful to measure the gene expression in primary mouse hepatocytes at two consecutive moments in time or at two different intervals. These moments should be chosen empirically depending on a suitable expression pattern of the genes listed in table 1 and 2 in the particular primary mouse hepatocytes chosen for the method. In general however, intervals of 1 to 2 days were found most appropriate. In the particular examples shown, it was chosen to analyse the gene expression at 24 hours and 48 hours after contacting the mouse hepatocytes with the test compound. This was found to produce very satisfying results.

In order to obtain reproducible results, it was found advantageous to obtain at least three independent readings of the gene expression. Hence, the above steps may be repeated at least twice to obtain a more reproducible and reliable result.

The results of the gene expression analysis may then be fed into a computer program capable of performing a supervised clustering analysis. This method was found to provide superior results as compared to unsupervised clustering methods and hierarchical clustering methods.

Supervised learning methods are computational approaches for class prediction based on biological data, such as generated with microarrays. Several methods have been shown to perform well with microarray data. Examples are support vector machines (SVM), k-nearest neighbours (KNN), diagonal linear discriminant analysis (DLDA), shrunken centroids (PAM), classification and regression trees (CART), probabilistic neural network (PNN) and Weighted Voting (WV). The shrunken centroids software (Prediction Analysis of Microarray, PAM, Version 2.1 (Sep 14, 2005), http://www-stat.stanford.edu/∼tibs/PAM/; Tibshirani et al. PNAS 2002 99:6567-6572) was used in this invention for identifying genes.

Such computer programs may be trained with a data set obtained for known true GTX and false GTX compounds at the intervals chosen, such as presented in Tables 5-8. These programs, when trained with a suitable data set, can be used to predict whether a compound is false GTX or true GTX. This is based on a computational comparison of expression of said at least one gene with and without the test compound at the two consecutive moment in time with data from reference compounds (e.g. provided in Table 5-8) by means of a supervised clustering method.

By repeating the steps of treating cells with the compound at least 2 times to obtain at least three measurements, at least six independent preliminary predictions can be obtained for the genotoxicity of a test compound; 3 repeats at two consecutive moments in time. These data may then be converted into a final prediction for the genotoxicity of a test compound by using the algorithm provided in table 3.

**Table 3**

| **Prediction at first time point** | **Prediction at second time point** | **Final prediction** |
|---|---|---|
| 3 repeats False positive | 3 repeats False positive | False-positive genotoxic = Not Genotoxic *in vivo* |
| 3 repeats False positive | 2 repeats False positive | False-positive genotoxic = Not Genotoxic *in vivo* |
| 3 repeats False positive | 1 repeats False positive | Equivocal = no prediction possible yet |
| 3 repeats False positive | 0 repeats False positive | True genotoxic = Genotoxic *in vivo* |
| 2 repeats False positive | 3 repeats False positive | False-positive genotoxic = Not Genotoxic *in vivo* |
| 2 repeats False positive | 2 repeats False positive | False-positive genotoxic = Not Genotoxic *in vivo* |
| 2 repeats False positive | 1 repeats False positive | Equivocal = no prediction possible yet |
| 2 repeats False positive | 0 repeats False positive | True genotoxic = Genotoxic *in vivo* |
| 1 repeats False positive | 3 repeats False positive | Equivocal = no prediction possible yet |
| 1 repeats False positive | 2 repeats False positive | Equivocal = no prediction possible yet |
| 1 repeats False positive | 1 repeats False positive | True genotoxic = Genotoxic *in vivo* |
| 1 repeats False positive | 0 repeats False positive | True genotoxic = Genotoxic *in vivo* |
| 0 repeats False positive | 3 repeats False positive | True genotoxic = Genotoxic *in vivo* |
| 0 repeats False positive | 2 repeats False positive | True genotoxic = Genotoxic *in vivo* |
| 0 repeats False positive | 1 repeats False positive | True genotoxic = Genotoxic *in vivo* |
| 0 repeats False positive | 0 repeats False positive | True genotoxic = Genotoxic *in vivo* |

Hence, the invention relates to a method for predicting whether a compound is a true in vivo genotoxic compound comprising the steps of
a. providing primary mouse hepatocytes
b. providing an assay capable of determining the expression of at least one gene selected from the group consisting of the genes provided in table 1 and table 2,
c. contacting the primary mouse hepatocytes with a test compound
d. Isolating RNA from said mouse hepatocytes at two consecutive moments in time and
e. determining the expression of at least one gene selected from the group consisting of the genes provided in table 1 and table 2,
f. repeating steps a to e at least 2 times to obtain at least three measurements of expression of said at least one gene
g. comparing the at least 3 measurements of expression of said at least one gene with and without the test compound at the two consecutive moment in time with data obtained from known true genotoxic compounds by means of a supervised clustering method,
h. obtaining at least 6 preliminary predictions for genotoxicity of the compound
i. predicting the true in vivo genotoxicity of the test compound according to the schedule of table 3.

The method according to the invention may even be improved by analyzing more than one gene selected from the group consisting of the genes provided in table 1 and table 2, such as 2 genes or more than 2, such as 3, 4, 5, 6, 7, 8, 9, 10, 11, 12, 13, 14, 15, 16, 17, 18, 19, 20, 21, 22, 23, 24, 25, 26, 27, 28, 29 or even 30 or more genes selected from the genes provided in table 1 and table 2.

In a method according to the invention, at least 3 measurements of expression of said at least one gene with and without the test compound at the two consecutive moment in time are compared with data obtained from known true genotoxic compounds. The true GTX compounds used in the study as presented here are known genotoxic compounds as well as known carcinogens. They exhibit a positive result in several in vitro assays as well as several in vivo models (Table 4). They are known to induce genotoxicity in vivo.

The false GTX compounds used in the present study are known non-genotoxic compounds in vivo as well as non-carcinogens in vivo. They only show a (false) positive result in certain in vitro tests, but are known not to induce genotoxicity in vivo. These compounds are also listed in table 4.

**Table 4. Overview of the compounds used in primary mouse hepatocyte exposure**

| **Chemical** | **Abbreviation** | **CAS nr.** |
|---|---|---|
| | | |
| *True GTX compounds* | | |
| Benzo(a)pyrene | BaP | 50-32-8 |
| Aflatoxin B1 | AFB1 | 1162-65-8 |
| 2-Acetylaminofluorene | 2-AAF | 53-96-3 |
| Dimethylnitrosamine | DMN | 62-75-9 |
| Mitomycin C | MitC | 50-07-7 |
| | | |
| *False GTX compounds* | | |
| o-Anthranilic acid | ANAC | 118-92-3 |
| 2-(Chloromethyl)pyridine.HCl | 2-CP | 6959-47-3 |
| 4-Nitro-o-phenylenediamine | 4-NP | 99-56-9 |
| Quercetin | Q | 117-39-5 |
| 8-Hydroxyquinoline | 8-HQ | 148-24-3 |

For the combination of the particular mouse hepatocytes and time intervals chosen in the study exemplified in the examples, the data obtained with the true GTX compounds at 24 and 48 hours are provided in table 5 and table 6 respectively. The corresponding data obtained with the false GTX compounds is provided in table 7 and table 8 respectively.

**Table 5 Gene expression data at 24 hr with true GTX compounds.**

| **GENEBANK** | **AFB1** | **BaP** | **2-AAF** | **DMN** | **MitC** |
|---|---|---|---|---|---|
| **ACCESS** | **24 h** | **24 h** | **24 h** | **24 h** | **24 h** |
| **CODE** | **average** | **average** | **average** | **average** | **average** |
| AK010447 | -1,2553 | -1,0413 | -0,17513 | -1,0358 | -1,43853 |
| BB318221 | -1,0808 | -1,67917 | -0,41097 | -1,04093 | -2,70633 |
| BG261907 | -1,11937 | -1,3429 | -0,25017 | -1,3871 | -3,87647 |
| Y15910 | -1,88907 | -1,35203 | -0,36603 | -1,63633 | -2,81277 |
| AV095209 | -0,83977 | -0,58853 | 0,4403 | -1,00697 | -2,45413 |
| AK019979 | -2,53743 | -0,6154 | 0,324767 | -1,32373 | -3,31723 |
| BC016073 | -1,09967 | -0,6631 | -0,1464 | -1,24277 | -1,96257 |
| BB821363 | -1,35393 | -1,40327 | -0,1216 | -1,143 | -2,48487 |
| Al596632 | -1,9945 | -1,8362 | -0,0981 | -1,90023 | -4,79467 |
| AW986246 | -0,33847 | 0,1617 | 0,018533 | -0,37203 | -0,21193 |
| NM_028803 | -1,00427 | -0,54163 | -0,2716 | -1,10333 | -1,8898 |
| AV141095 | -1,29513 | -1,05753 | -0,28807 | -1,37477 | -2,28737 |
| AF000969 | -2,16997 | -1,90953 | -0,53713 | -1,03907 | -2,40647 |
| BB526605 | -0,7181 | -0,7419 | 0,346567 | -1,5816 | -2,03513 |
| NM_008576 | -0,18853 | 0,1024 | 0,332067 | -0,92953 | -1,31177 |
| BG070887 | -1,47347 | -0,97337 | -0,01607 | -1,11183 | -2,35577 |
| AW543460 | -0,2756 | -0,61937 | -0,3156 | -1,4802 | -3,16427 |
| BC016265 | -0,87973 | -0,75103 | -0,39843 | -1,28743 | -2,20177 |
| AV223474 | -1,02503 | -1,37517 | -0,44587 | -0,9067 | -2,28283 |
| Al987929 | -0,8074 | -0,36477 | 0,8432 | -0,98383 | -3,32443 |
| AK009736 | -0,50757 | 0,217433 | -0,00723 | -0,83673 | -1,05647 |
| AK007766 | -1,55373 | -1,28833 | 0,010033 | -0,8706 | -2,22737 |
| AK004419 | -1,36467 | -1,00123 | -0,16797 | -1,22027 | -4,0931 |
| AV173571 | -1,23913 | -1,12323 | -0,04133 | -1,0106 | -2,28717 |
| BB308836 | -1,07683 | -0,61223 | -0,14993 | -0,98813 | -1,64007 |
| BC004827 | -1,0032 | -0,04097 | 0,279733 | -1,51817 | -2,4733 |
| AW240761 | -1,11773 | -0,703 | -0,0924 | -1,19117 | -2,64317 |
| BG066903 | -0,50743 | -0,13297 | -0,21247 | -0,86817 | -1,04667 |
| NM_025770 | -1,12757 | -1,05323 | 0,016633 | -0,51127 | -1,32903 |
| BC025915 | -0,64197 | -0,53963 | -0,15683 | -0,95017 | -1,61897 |
| NM_018770 | -0,43897 | -0,8982 | -0,20653 | -0,84117 | -2,05413 |
| AF022072 | -1,2526 | -0,28443 | 0,5527 | -1,91293 | -3,6802 |
| BC025837 | 0,469433 | 0,380233 | -0,2976 | 0,9087 | 1,652233 |
| BG076151 | 0,602567 | 0,727967 | -0,12193 | 1,946333 | 2,237733 |
| BF719766 | 0,389167 | 0,9747 | -0,0442 | 1,823667 | 2,045133 |
| AV377066 | 1,0883 | 0,757233 | -0,65863 | 0,960533 | 2,462133 |
| BG065754 | 0,838267 | 0,8607 | 0,0569 | 0,958667 | 1,179133 |
| BC025501 | 1,140267 | 1,5389 | -0,1475 | 1,6386 | 2,022933 |
| NM_134188 | 0,012067 | 0,5856 | 0,596967 | -0,50007 | -0,06593 |
| NM_021451 | 1,100433 | 0,6364 | -0,7054 | 3,698367 | 2,681033 |
| BC026422 | 0,8843 | 0,6019 | -0,16643 | 2,220467 | 2,651367 |
| BC015270 | 1,092667 | 0,790767 | -0,13273 | 0,981567 | 0,633867 |
| NM_053168 | 1,268333 | 0,823033 | -0,26757 | 1,0583 | 1,4821 |
| BB027848 | -0,016 | 0,0761 | -0,252 | 1,126333 | 2,179833 |
| AV327248 | 0,805533 | 1,102433 | 0,0668 | 4,114233 | 4,258133 |
| AV219418 | 0,281667 | 0,278133 | -0,36947 | 2,743333 | 2,872767 |
| BG069873 | 0,727567 | 1,299367 | -0,27473 | 2,2987 | 2,174733 |
| AF204959 | 1,403933 | 0,3057 | 0,2152 | -0,29927 | 0,8169 |
| NM_030697 | 1,8143 | 1,4578 | -0,25197 | 3,210733 | 3,874367 |
| BM198879 | 1,610067 | 1,054033 | -0,2382 | 1,2634 | 2,011933 |
| AW543723 | 1,426867 | 1,993167 | 0,106467 | 1,950433 | 2,3145 |
| AK014608 | 1,764733 | 1,664767 | -0,09823 | 1,756333 | 2,3711 |
| AV298304 | 1,030433 | 0,6204 | -0,18307 | 1,8715 | 1,9706 |
| BC012260 | 1,2375 | 0,312733 | -0,64603 | 1,0548 | 2,236967 |
| NM_013866 | 0,951267 | 0,776033 | -0,13603 | 2,361133 | 2,300333 |
| AF065917 | 0,689633 | 0,922733 | -0,12417 | 1,803833 | 1,033367 |
| AF297615 | 1,1224 | 1,734833 | 0,031867 | 3,2188 | 2,358333 |
| BB770528 | 0,7477 | 0,697733 | -0,5052 | 2,799567 | 2,603367 |
| BC012247 | 0,367667 | 0,445067 | -0,08253 | 1,6567 | 2,093633 |
| NM_011316 | 0,2733 | 0,187133 | -0,3157 | 2,1585 | 2,611367 |
| NM_007987 | 0,627967 | 0,828667 | 0,069233 | 2,420733 | 2,6645 |
| B1660702 | 0,4068 | 1,3867 | 0,1219 | 2,513867 | 3,741633 |
| BM230508 | 0,591867 | 1,297 | 0,282033 | 2,303567 | 2,556067 |
| Al594683 | 1,047633 | 0,731 | -0,24517 | 3,163767 | 3,670633 |
| NM_011176 | 1,288967 | 1,507833 | -0,03387 | 1,574367 | 2,077667 |
| BB463610 | 1,7658 | 2,143967 | -0,37777 | 1,9965 | 2,535 |
| BC019882 | 0,4654 | 2,0133 | 1,3218 | 2,057333 | 3,446367 |
| AK007854 | 1,391233 | 0,3514 | -0,90083 | 2,172867 | 2,898333 |
| BC010462 | 0,998467 | 0,666267 | -0,14753 | 0,751867 | 1,162867 |
| BB043558 | 1,671433 | 1,246667 | -0,12047 | 2,406733 | 2,307633 |
| NM_008522 | 1,054933 | 1,113967 | 0,127667 | 3,616333 | 3,9908 |
| NM_012006 | 1,250567 | 3,254667 | 1,9403 | 2,032633 | 3,958567 |
| BB275142 | 1,009433 | 1,003367 | -0,02303 | 1,902233 | 1,944367 |
| BC008626 | 1,4158 | 0,823433 | -0,63527 | 2,035033 | 2,3005 |
| Bl651416 | 1,8011 | 1,678867 | 0,155167 | 1,7218 | 2,482133 |
| AK005731 | 2,2462 | 2,015867 | 0,082767 | 5,486167 | 5,7645 |

**Table 6 Gene expression data at 24 hr with false GTX compounds**

| **GENEBANK** | **2-CP** | **4-NP** | **ANAC** | **Q** | **8Q** |
|---|---|---|---|---|---|
| **ACCESS** | **24 h** | **24 h** | **24 h** | **24 h** | **24 h** |
| **CODE** | **average** | **average** | **average** | **average** | **average** |
| AK010447 | 0,060966667 | 0,496666667 | -0,121066667 | 0,0701 | 0,076966667 |
| BB318221 | -0,2464 | 0,0942 | 0,000233333 | -0,147933333 | 0,113766667 |
| BG261907 | -0,117733333 | 0,3643 | 0,092933333 | 0,3964 | -0,1486 |
| Y15910 | 0,066933333 | -0,539833333 | 0,065333333 | -0,310033333 | -0,2692 |
| AV095209 | 0,5754 | 0,907233333 | 0,316166667 | 0,8331 | 0,156433333 |
| AK019979 | 0,3731 | 0,938366667 | 0,113866667 | 0,250166667 | 0,084466667 |
| BC016073 | 0,030166667 | 0,362733333 | 0,0051 | 0,199266667 | -0,051833333 |
| BB821363 | -0,188333333 | -0,218966667 | -0,084 | -0,233766667 | 0,1765 |
| Al596632 | 0,225066667 | -0,496166667 | 0,1214 | -0,456033333 | -0,226966667 |
| AW986246 | 1,210366667 | 1,7055 | 0,016833333 | 1,388133333 | 0,319833333 |
| NM_028803 | 0,524233333 | 0,363333333 | -0,0826 | -0,250933333 | -0,045933333 |
| AV141095 | 0,181666667 | 0,178933333 | -0,129333333 | 0,285466667 | -0,070466667 |
| AF000969 | -0,100266667 | -0,683866667 | -0,12 | -0,575266667 | -0,371733333 |
| BB526605 | -0,213366667 | 1,641133333 | 0,340733333 | 0,096166667 | -0,014533333 |
| NM_008576 | 1,199266667 | 1,696333333 | 0,358566667 | 1,2925 | 0,106 |
| BG070887 | -0,067733333 | 0,343166667 | -0,051633333 | -0,2209 | -0,042733333 |
| AW543460 | 0,226333333 | 0,912666667 | 0,060366667 | 0,243666667 | -0,0366 |
| BC016265 | 0,0177 | 0,034333333 | 0,015066667 | 0,162166667 | -0,254666667 |
| AV223474 | -0,409433333 | -0,084366667 | -0,073466667 | -0,2496 | -0,069566667 |
| Al987929 | -0,100433333 | 2,650333333 | 0,6977 | 1,662633333 | 0,441433333 |
| AK009736 | 0,9644 | 1,582066667 | -0,0957 | 1,170733333 | 0,533733333 |
| AK007766 | -0,123533333 | -0,169666667 | -0,051666667 | -0,044633333 | -0,0226 |
| AK004419 | -0,120266667 | -0,0775 | -0,017933333 | -0,015366667 | 0,011133333 |
| AV173571 | -0,013666667 | -0,003133333 | -0,157433333 | -0,248433333 | 0,041966667 |
| BB308836 | -0,369233333 | 0,037633333 | -0,0317 | 0,1666 | 0,2558 |
| BC004827 | 0,365366667 | 0,539466667 | 0,393733333 | 0,9258 | -0,101133333 |
| AW240761 | 0,028033333 | 0,026966667 | 0,001666667 | -0,157033333 | -0,200633333 |
| BG066903 | 0,336833333 | 1,004933333 | 0,0241 | 0,250566667 | -0,1277 |
| NM_025770 | 0,344466667 | 1,050666667 | -0,012633333 | 0,266333333 | -0,1372 |
| BC025915 | 0,058266667 | 0,3343 | -0,053833333 | 0,317566667 | -0,026433333 |
| NM_018770 | 0,139966667 | 0,386766667 | -0,088533333 | 0,468866667 | 0,1391 |
| AF022072 | 0,621 | 0,848566667 | 0,4544 | 0,3385 | -0,4275 |
| BC025837 | -0,504233333 | -0,818133333 | -0,3033 | -0,3383 | -0,042266667 |
| BG076151 | 0,121266667 | -0,2933 | -0,086366667 | -0,136733333 | 0,072433333 |
| BF719766 | 0,0395 | -0,2859 | -0,115266667 | 0,159566667 | -0,193466667 |
| AV377066 | -0,6354 | -1,694833333 | -0,2482 | -0,4922 | -0,321166667 |
| BG065754 | 0,3287 | -0,250333333 | -0,089166667 | 0,482866667 | -0,2279 |
| BC025501 | 0,0663 | -0,2167 | -0,226066667 | 0,4589 | 0,197866667 |
| NM_134188 | 0,501033333 | -0,763733333 | 0,869566667 | 0,127533333 | -0,2881 |
| NM_021451 | 0,023566667 | -1,505 | 0,013033333 | -0,611033333 | -0,529466667 |
| BC026422 | -0,3825 | -0,8176 | 0,0232 | 0,280033333 | 0,287266667 |
| BC015270 | -0,230966667 | -0,7846 | 0,118566667 | -0,392366667 | -0,033533333 |
| NM_053168 | -0,067833333 | -0,1598 | -0,201 | -0,022566667 | -0,0493 |
| BB027848 | -0,571633333 | -1,7858 | -0,036966667 | -1,303666667 | -0,280733333 |
| AV327248 | 0,147666667 | -0,089166667 | 0,0212 | 0,311566667 | -0,082 |
| AV219418 | -0,796766667 | -1,032 | -0,2907 | -0,180833333 | 0,0363 |
| BG069873 | -0,1027 | -0,132566667 | -0,210733333 | 0,007866667 | -0,047633333 |
| AF204959 | -0,5019 | -2,025133333 | -0,280033333 | -1,232033333 | -0,417833333 |
| NM_030697 | -0,578533333 | 0,181633333 | -0,162333333 | 1,3142 | 0,220433333 |
| BM198879 | 0,293533333 | -0,328833333 | -0,2194 | 0,1402 | -0,006766667 |
| AW543723 | 0,148666667 | 0,670633333 | 0,294266667 | 0,419266667 | -0,0994 |
| AK014608 | 0,5726 | 0,295766667 | -0,176833333 | 0,032733333 | -0,122533333 |
| AV298304 | 0,1881 | -0,583933333 | -0,0861 | 0,2496 | -0,036066667 |
| BC012260 | -0,824033333 | 0,118933333 | -0,1838 | -0,168866667 | 0,254466667 |
| NM_013866 | -0,1346 | -0,280766667 | -0,195333333 | 0,065433333 | 0,058933333 |
| AF065917 | 0,012133333 | -0,457266667 | -0,202333333 | -0,102433333 | -0,210133333 |
| AF297615 | 0,069633333 | -0,279733333 | 0,031033333 | 0,804666667 | -0,0684 |
| BB770528 | -0,327066667 | -1,122266667 | -0,083266667 | -0,310933333 | 0,138566667 |
| BC012247 | -0,326066667 | -1,499833333 | -0,0311 | -0,2845 | -0,0122 |
| NM_011316 | -0,044633333 | -0,590933333 | -0,1988 | -0,131366667 | 0,0831 |
| NM_007987 | 0,231266667 | -1,231166667 | -0,133733333 | 0,174366667 | -0,055233333 |
| Bl660702 | 0,081666667 | -0,608966667 | -0,153866667 | -0,058966667 | 0,051133333 |
| BM230508 | 0,3615 | -0,6065 | -0,1178 | 0,3449 | 0,034966667 |
| Al594683 | -0,315666667 | -0,351733333 | -0,216266667 | -0,2712 | -0,097833333 |
| NM_011176 | -0,053933333 | -0,571666667 | -0,144533333 | 0,591366667 | 0,057833333 |
| BB463610 | 0,384833333 | 0,210633333 | -0,3672 | -0,1026 | -0,2188 |
| BC019882 | -0,0399 | -1,259166667 | 1,0984 | 0,712366667 | 0,0605 |
| AK007854 | -0,4492 | -1,735733333 | -0,428266667 | -0,9336 | -0,076 |
| BC010462 | -0,589533333 | -0,865433333 | -0,408866667 | -0,5852 | 0,056633333 |
| BB043558 | 0,494633333 | -0,415933333 | -0,129166667 | 0,343133333 | -0,373433333 |
| NM_008522 | -0,358133333 | -0,301766667 | 0,0771 | -0,095933333 | -0,059633333 |
| NM_012006 | 0,6012 | -1,4501 | 1,861966667 | 1,0762 | -0,253166667 |
| BB275142 | -0,195366667 | -0,418433333 | 0,028233333 | -0,500433333 | -0,025066667 |
| BC008626 | -0,242 | -2,247633333 | -0,270533333 | -0,372466667 | -0,4422 |
| Bl651416 | 0,052366667 | -0,0339 | 0,013866667 | 0,265266667 | 0,136366667 |
| AK005731 | 0,196466667 | -0,511133333 | -0,173333333 | 0,6919 | 0,114266667 |

**Table 7 Gene expression data at 48 hr with true GTX compounds**

| **GENEBANK** | **AFB1** | **BaP** | **2-AAF** | **DMN** | **MitC** |
|---|---|---|---|---|---|
| **ACCESS** | **48 h** | **48 h** | **48 h** | **48 h** | **48 h** |
| **CODE** | **average** | **average** | **average** | **average** | **average** |
| AK005731 | 2,2191 | 3,0498 | 0,011133333 | 4,616833333 | 6,088666667 |
| Bl651416 | 1,978966667 | 1,9573 | 0,350166667 | 1,8588 | 2,2069 |
| NM_008522 | 2,271133333 | 2,9351 | 0,1967 | 4,993466667 | 5,9249 |
| BB043558 | 1,629366667 | 1,692466667 | 0,195966667 | 2,186833333 | 2,331833333 |
| NM_007987 | 0,877166667 | 1,352666667 | 0,515966667 | 1,665166667 | 2,2406 |
| BC022148 | 1,0426 | 1,323566667 | 0,456033333 | 1,8449 | 2,668233333 |
| BC019882 | 1,2329 | 2,160033333 | 1,576933333 | 1,494 | 2,441633333 |
| BB463610 | 1,2472 | 1,672366667 | 0,0872 | 1,546 | 2,727766667 |
| BM230508 | 0,637 | 1,158366667 | 0,0745 | 1,539833333 | 2,570533333 |
| Al594683 | 1,508466667 | 1,465666667 | -0,052133333 | 3,7761 | 4,611033333 |
| AV327248 | 1,4639 | 2,067166667 | -0,091766667 | 4,022733333 | 4,918266667 |
| BE956581 | 1,7703 | 1,451433333 | 0,1808 | 3,032866667 | 3,679666667 |
| NM_011176 | 1,3047 | 1,4287 | -0,263233333 | 1,597133333 | 1,856466667 |
| BM200015 | 0,934166667 | 1,0174 | 0,512666667 | 1,160333333 | 1,702233333 |
| BB223872 | 0,526333333 | 1,1634 | 0,321566667 | 1,694233333 | 2,200766667 |
| AF297615 | 2,175266667 | 1,571833333 | -0,679133333 | 3,153166667 | 1,840933333 |
| BC027026 | 0,6995 | 1,378666667 | 0,806333333 | 2,570766667 | 2,873 |
| NM_012006 | 1,7541 | 2,867133333 | 2,770866667 | 1,6671 | 3,166133333 |
| AK014608 | 1,185766667 | 1,245366667 | -0,019866667 | 1,390533333 | 2,8746 |
| BC012247 | 0,7954 | 1,476733333 | 0,6286 | 1,669566667 | 2,2674 |
| BC027121 | 0,979866667 | 1,345266667 | -0,134133333 | 2,074533333 | 2,741666667 |
| BG797099 | 1,027266667 | 1,8809 | -0,061733333 | 1,703866667 | 2,6244 |
| BB743970 | 0,381333333 | 1,510133333 | 0,766433333 | 3,154366667 | 3,5588 |
| BF719766 | 1,1094 | 0,950666667 | 0,265166667 | 1,2016 | 2,112066667 |
| BC027185 | 0,0776 | 0,708133333 | 0,4588 | 1,461733333 | 2,115066667 |
| AF033112 | 1,032833333 | 1,4254 | -0,039466667 | 1,816933333 | 2,218866667 |
| BG065754 | 0,688433333 | 0,974633333 | 0,373833333 | 0,995333333 | 1,3079 |
| BB781615 | 0,972233333 | 0,927133333 | -0,116433333 | 1,486966667 | 0,895166667 |
| BC013893 | 0,3479 | 1,040066667 | 0,493633333 | 0,548733333 | 1,863933333 |
| BC003284 | 0,703633333 | 0,731333333 | 0,025633333 | 1,379133333 | 2,092633333 |
| BC006713 | 0,941966667 | 0,4721 | 0,4411 | 1,668066667 | 1,2958 |
| NM_011075 | 1,1557 | 1,197733333 | -0,5522 | 2,401 | 3,096466667 |
| BB009155 | 1,3686 | 0,784 | -0,5851 | 2,342966667 | 2,9201 |
| BG967046 | 0,624866667 | 0,593566667 | 0,0391 | 1,1973 | 1,305133333 |
| NM_030697 | 1,270633333 | 1,3687 | -0,478333333 | 2,5583 | 3,958066667 |
| BB275142 | 0,5302 | 1,0559 | 0,110166667 | 1,161633333 | 2,281633333 |
| AV246296 | 1,097933333 | 1,1524 | -0,54 | 0,957233333 | 1,4788 |
| NM_013738 | 0,4146 | 0,9012 | -0,239566667 | 1,562466667 | 2,749033333 |
| NM_018881 | 0,200133333 | 3,369333333 | 0,906066667 | 0,948066667 | 2,279366667 |
| BM936480 | 0,1463 | 3,0213 | 0,916866667 | 0,778066667 | 2,006033333 |
| BM198879 | 1,579166667 | 0,808866667 | -0,288733333 | 1,096266667 | 1,927533333 |
| AK018383 | 0,406933333 | 1,273266667 | 0,230266667 | 0,824633333 | 0,994 |
| AV254764 | 1,542533333 | 1,480466667 | -0,006466667 | 1,133466667 | 1,411166667 |
| BC021352 | 1,590266667 | 0,705233333 | -1,126533333 | 3,512033333 | 3,121333333 |
| BB027848 | -0,004366667 | 0,473766667 | 0,5378 | 1,190666667 | 1,879 |
| AK017734 | 0,4796 | 0,7529 | 0,0451 | 1,0611 | 1,834766667 |
| AF069954 | 0,2361 | 0,8863 | 0,351766667 | 1,631166667 | 2,259766667 |
| BB770528 | 0,867766667 | 0,7877 | -0,5594 | 1,709333333 | 2,0332 |
| NM_009897 | 1,053566667 | 1,182233333 | -0,003733333 | 3,618966667 | 4,658266667 |
| AK007854 | 1,5416 | 1,6125 | 1,4303 | 0,839633333 | 1,500233333 |
| Bl966443 | 0,698966667 | 1,3186 | 0,1201 | 3,294933333 | 4,094033333 |
| NM_013929 | 0,744866667 | 1,061633333 | -0,041633333 | 1,890866667 | 2,481333333 |
| BG076151 | 0,477866667 | 0,759933333 | -0,3358 | 1,205166667 | 2,5119 |
| AV251625 | 0,547133333 | 1,730566667 | 0,071033333 | 1,246066667 | 2,476566667 |
| AV219418 | 0,8603 | 1,558433333 | 0,783933333 | 4,5473 | 4,167766667 |
| NM_011316 | 0,6494 | 0,873466667 | 0,4369 | 2,0898 | 2,255733333 |
| NM_007980 | 0,411966667 | 1,752833333 | 1,503166667 | 0,325166667 | 2,9391 |
| BB046347 | 0,5079 | 0,7766 | 0,217766667 | 0,774333333 | 1,349533333 |
| AF335325 | 1,3645 | 1,4875 | -0,308833333 | 1,6857 | 1,754566667 |
| AK010738 | 0,6579 | 0,911166667 | 0,1851 | 1,342566667 | 1,979733333 |
| NM_134188 | -0,158966667 | 0,288366667 | 0,908766667 | -0,637766667 | 0,4786 |
| NM_008935 | -1,772 | -1,4799 | 0,027833333 | -1,382066667 | -3,600866667 |
| BB140436 | -0,4938 | -0,457233333 | 0,469333333 | -1,281166667 | -1,601366667 |
| NM_019738 | -2,369733333 | -2,585566667 | -0,193033333 | -2,9169 | -2,771766667 |
| X62701 | -0,661133333 | -1,2338 | -0,356033333 | 0,071566667 | -1,3159 |
| AV141095 | -1,192433333 | -1,4056 | -0,582933333 | -0,866333333 | -2,411233333 |
| Al747296 | -1,533166667 | -2,124066667 | -0,500266667 | -0,433266667 | -2,056266667 |
| BC005552 | -0,7309 | -0,212133333 | -0,125966667 | -0,523533333 | -1,341933333 |
| BB458460 | -1,131533333 | -1,384133333 | -0,336766667 | -0,693433333 | -2,7207 |
| BG076333 | -0,3573 | -0,409133333 | -0,130566667 | -0,799466667 | -1,870033333 |
| AK019979 | -2,270633333 | -1,0966 | -0,509866667 | -1,110233333 | -3,757366667 |
| AV095209 | -0,6169 | -0,765433333 | -0,371833333 | -0,453966667 | -2,780333333 |
| AV216768 | -1,281733333 | -1,446966667 | -0,6814 | -0,2641 | -4,148766667 |
| AV221299 | -0,787333333 | -1,168933333 | 0,6759 | -1,5693 | -3,709666667 |
| BQ174991 | -0,6262 | -1,407933333 | -0,342633333 | -0,28 | -2,777066667 |
| NM_013642 | -0,252766667 | -1,442166667 | -0,653466667 | 0,3461 | -0,784533333 |
| L21027 | -1,867666667 | -1,779266667 | -0,728966667 | -0,4472 | -4,611766667 |
| BB204486 | -1,371333333 | -1,5693 | -0,6445 | -0,3108 | -4,143533333 |
| BC025169 | -1,029533333 | -0,511366667 | -0,222233333 | -1,295533333 | -3,296166667 |
| BC026131 | -0,447566667 | -1,045333333 | -0,3489 | -0,7571 | -0,998633333 |
| BC010318 | -0,956533333 | -0,552866667 | -0,022133333 | -1,166866667 | -1,728666667 |
| BB730977 | -0,256 | 0,421766667 | -0,993666667 | -0,656 | -2,360033333 |
| AA561726 | -1,480766667 | -1,7432 | -0,7419 | -0,353433333 | -4,371633333 |
| BC012955 | -1,402833333 | -0,944466667 | 0,050733333 | -1,970133333 | -2,240533333 |
| BC004827 | -1,254533333 | -1,186033333 | -0,2015 | -1,0689 | -3,638633333 |
| NM_007556 | -0,9667 | -1,089 | 0,027033333 | -1,289533333 | -4,012333333 |
| NM_134147 | -1,510133333 | -1,2396 | 0,042133333 | -1,579366667 | -2,8471 |
| AV173869 | -0,881433333 | -1,923533333 | -0,3782 | -1,271666667 | -2,2889 |
| AF022072 | -0,991333333 | -1,017566667 | 0,0482 | -1,2797 | -3,369966667 |
| BC019379 | -1,263466667 | -1,9807 | -1,214833333 | -0,360166667 | -2,7493 |
| AK010447 | -1,2768 | -1,207066667 | -0,3256 | -0,863433333 | -1,6388 |
| BC017615 | -2,585433333 | -2,233833333 | -0,522933333 | -2,7334 | -3,946966667 |
| BB246912 | -1,2439 | -1,539866667 | -0,226366667 | -0,980566667 | -1,866433333 |
| AF000969 | -2,345533333 | -2,251433333 | -0,831866667 | -1,113833333 | -3,3606 |
| BG066491 | -1,6995 | -2,097833333 | -1,541033333 | -0,975666667 | -1,928533333 |
| AF055573 | -2,3744 | -1,5573 | -0,289466667 | -1,912533333 | -2,915766667 |
| NM_053122 | -2,304433333 | -1,795033333 | -0,105966667 | -2,3248 | -4,049166667 |

**Table 8 Gene expression data at 48 hr with false GTX compounds**

| **GENEBANK** | **2-CP** | **4-NP** | **ANAC** | **Q** | **8Q** |
|---|---|---|---|---|---|
| **ACCESS** | **48 h** | **48 h** | **48 h** | **48 h** | **48 h** |
| **CODE** | **average** | **average** | **average** | **average** | **average** |
| AK005731 | -0,6019 | -0,9581 | -0,649766667 | -0,8953 | -0,039966667 |
| Bl651416 | -0,087533333 | -0,028733333 | -0,105066667 | -0,498166667 | 0,067766667 |
| NM_008522 | 0,0213 | -0,239266667 | -0,069566667 | -0,039166667 | 0,0486 |
| BB043558 | -0,229266667 | -0,273633333 | -0,188 | 0,038166667 | -0,234666667 |
| NM_007987 | -0,3447 | -0,8469 | -0,005833333 | -0,073433333 | 0,034866667 |
| BC022148 | 0,191933333 | -1,1322 | -0,1976 | 0,2323 | 0,044966667 |
| BC019882 | 0,814833333 | -2,4942 | 0,194833333 | -0,021766667 | -0,171866667 |
| BB463610 | 0,0113 | -0,037266667 | -0,218566667 | -0,254 | -0,236866667 |
| BM230508 | -0,2554 | -0,973133333 | -0,470966667 | -0,670733333 | 0,076033333 |
| Al594683 | -0,2197 | -0,4763 | -0,522366667 | -0,1952 | 0,123533333 |
| AV327248 | -0,221533333 | -0,014933333 | 0,0856 | -0,184166667 | 0,117633333 |
| BE956581 | 0,0444 | 0,0346 | 0,042666667 | 0,106333333 | 0,403033333 |
| NM_011176 | -0,0046 | -1,635266667 | -0,460666667 | -0,357866667 | 0,1358 |
| BM200015 | -0,3607 | -0,4429 | 0,009533333 | -0,2469 | 0,080866667 |
| BB223872 | 0,174333333 | -0,653566667 | -0,428733333 | -0,240866667 | 0,057533333 |
| AF297615 | -0,5906 | -1,352433333 | 0,111 | -0,029433333 | -0,009333333 |
| BC027026 | -0,217666667 | 0,052733333 | -0,007933333 | 0,363633333 | 0,2479 |
| NM_012006 | -0,1277 | -0,006733333 | 1,5971 | 0,737333333 | 0,580566667 |
| AK014608 | -0,012633333 | -0,307233333 | -0,455 | -0,318133333 | -0,2447 |
| BC012247 | 0,522066667 | -0,635466667 | -0,5319 | 0,185266667 | -0,006933333 |
| BC027121 | -0,6553 | -0,2406 | -0,1424 | 0,003766667 | -0,1207 |
| BG797099 | 0,004233333 | -0,066966667 | -0,020966667 | -0,318533333 | 0,0658 |
| BB743970 | -0,168566667 | -0,1636 | 0,155133333 | -0,154266667 | 0,1936 |
| BF719766 | -0,125866667 | -0,5024 | 0,076366667 | 0,168333333 | -0,271666667 |
| BC027185 | 0,062733333 | -1,381733333 | -0,238133333 | -0,431666667 | -0,120933333 |
| AF033112 | -0,665066667 | 0,151966667 | 0,142233333 | 0,025933333 | -0,017033333 |
| BG065754 | -0,2817 | -0,338066667 | 0,0641 | -0,269166667 | -0,0909 |
| BB781615 | 0,007033333 | -0,750133333 | -0,293733333 | -0,302466667 | -0,006366667 |
| BC013893 | 0,487133333 | -1,496 | -0,5709 | -0,5246 | -0,165433333 |
| BC003284 | -0,239466667 | -0,587633333 | -0,210833333 | -0,3991 | 0,09 |
| BC006713 | 0,2405 | -0,7469 | -0,130466667 | 0,141933333 | -0,103533333 |
| NM_011075 | -0,578166667 | -1,007866667 | 0,389133333 | -0,8714 | 0,161766667 |
| BB009155 | -0,348033333 | -0,6079 | -0,4697 | -0,644766667 | 0,006466667 |
| BG967046 | -0,155666667 | -0,632366667 | -0,256166667 | -0,2263 | -0,092566667 |
| NM_030697 | -0,0882 | -0,3339 | -0,6238 | 0,085966667 | -0,0261 |
| BB275142 | 0,091 | -0,196266667 | -0,287366667 | -0,530666667 | 0,0112 |
| AV246296 | -0,355833333 | -1,045766667 | -0,1054 | -0,449 | -0,113566667 |
| NM_013738 | -0,373033333 | -1,171233333 | -0,3378 | -0,4434 | -0,304966667 |
| NM_018881 | -0,0599 | -0,2965 | 0,083833333 | 0,266733333 | -0,167866667 |
| BM936480 | -0,074166667 | -0,411966667 | -0,043233333 | 0,1682 | 0,0483 |
| BM198879 | -0,102566667 | -0,421266667 | -0,160833333 | -0,138233333 | -0,056166667 |
| AK018383 | -0,215766667 | -0,500633333 | -0,0203 | -0,063433333 | 0,0679 |
| AV254764 | 0,396 | -0,449933333 | -0,264966667 | -0,091133333 | 0,024033333 |
| BC021352 | -0,719166667 | -1,279566667 | 0,065633333 | -1,142833333 | 0,472066667 |
| BB027848 | 0,205366667 | -1,954166667 | -0,2609 | -0,5634 | -0,066066667 |
| AK017734 | 0,148866667 | -1,2326 | -0,527466667 | -0,223133333 | 0,0878 |
| AF069954 | 0,199 | -0,442866667 | -0,384666667 | -0,168 | 0,184133333 |
| BB770528 | -0,4836 | -0,9985 | -0,001733333 | -0,5238 | -0,018233333 |
| NM_009897 | -0,061033333 | -0,019033333 | 0,099633333 | 0,246833333 | 0,111033333 |
| AK007854 | 0,551333333 | -0,141066667 | -0,224233333 | 1,0645 | 0,059733333 |
| Bl966443 | -0,017133333 | 0,2259 | 0,077233333 | 0,1621 | 0,133533333 |
| NM_013929 | -0,529133333 | 0,175266667 | -0,006633333 | 0,092566667 | 0,033 |
| BG076151 | -0,0769 | -0,657266667 | -0,240966667 | -0,7006 | -0,188666667 |
| AV251625 | 0,067233333 | -0,1615 | 0,0423 | -0,2405 | 0,038133333 |
| AV219418 | 0,828066667 | 0,6295 | 0,153866667 | 0,7223 | 0,4492 |
| NM_011316 | 0,582866667 | -0,9295 | -0,471733333 | -0,248533333 | 0,063766667 |
| NM_007980 | -0,284766667 | -1,581066667 | 0,177566667 | 1,050633333 | -0,506433333 |
| BB046347 | 0,187 | -0,804466667 | -0,107166667 | 0,374733333 | 0,1608 |
| AF335325 | -0,056666667 | 0,507133333 | -0,0865 | -0,3547 | 0,141766667 |
| AK010738 | 0,0059 | -0,156866667 | -0,066866667 | 0,034566667 | 0,3608 |
| NM_134188 | -0,137266667 | -0,440166667 | 0,238166667 | 0,577266667 | -0,070666667 |
| NM_008935 | -0,212433333 | -1,001666667 | 0,377866667 | 0,588066667 | 0,408166667 |
| BB140436 | 0,2823 | 2,071 | -0,072366667 | 0,455033333 | 0,286066667 |
| NM_019738 | -0,514266667 | -1,132266667 | -0,632566667 | -0,7691 | -0,9543 |
| X62701 | -0,146033333 | 0,7376 | 0,7542 | 0,842633333 | 0,154 |
| AV141095 | -0,4102 | -0,105133333 | -0,0311 | -0,035733333 | -0,172533333 |
| Al747296 | -0,263733333 | -0,3288 | -0,0394 | -0,157233333 | -0,000966667 |
| BC005552 | 0,125466667 | 0,4521 | 0,305566667 | 0,365 | 0,134333333 |
| BB458460 | -0,179166667 | 0,164466667 | 0,007966667 | -0,250466667 | 0,131733333 |
| BG076333 | 0,4759 | 0,873133333 | 0,238266667 | 0,256933333 | -0,053333333 |
| AK019979 | -0,424933333 | 0,040866667 | 0,045466667 | -0,645633333 | -0,215433333 |
| AV095209 | -0,034666667 | 0,6413 | 0,2956 | 0,0573 | 0,260166667 |
| AV216768 | -0,417933333 | -0,004066667 | 0,415333333 | 0,4269 | 0,167866667 |
| AV221299 | 0,454466667 | 0,7477 | 0,164933333 | 0,3941 | 0,1454 |
| BQ174991 | 0,1864 | 0,401733333 | 0,4039 | 0,543166667 | -0,1546 |
| NM_013642 | 0,3967 | 1,007033333 | 0,328566667 | 0,7153 | 0,265866667 |
| L21027 | -0,5404 | -0,030733333 | 0,3857 | 0,4391 | 0,2905 |
| BB204486 | -0,3875 | -0,0837 | 0,3188 | 0,321133333 | 0,130466667 |
| BC025169 | 0,928266667 | 0,371166667 | -0,0788 | 0,176 | 0,022133333 |
| BC026131 | 0,4079 | 0,212366667 | 0,0392 | 0,3886 | -0,0852 |
| BC010318 | 0,1986 | 0,1861 | 0,135333333 | 0,227366667 | 0,0565 |
| BB730977 | -0,7313 | 0,991966667 | 0,753233333 | 0,126966667 | 0,329766667 |
| AA561726 | -0,429733333 | -0,08 | 0,319366667 | 0,347333333 | 0,173933333 |
| BC012955 | 0,2863 | 0,105133333 | -0,255433333 | 0,249966667 | -0,304933333 |
| BC004827 | -0,247666667 | -0,2247 | 0,375566667 | 0,383233333 | -0,070433333 |
| NM_007556 | 0,168533333 | 0,427366667 | 0,524666667 | 0,467266667 | 0,0638 |
| NM_134147 | 0,6449 | 0,014333333 | -0,220733333 | -0,2048 | -0,1757 |
| AV173869 | 0,3109 | 0,1411 | -0,005066667 | -0,3593 | -0,025566667 |
| AF022072 | 0,040666667 | 0,606166667 | 0,5563 | 0,7799 | 0,0814 |
| BC019379 | -0,192566667 | -0,5665 | 0,0072 | -0,033133333 | -0,155466667 |
| AK010447 | -0,050233333 | 0,595633333 | -0,108333333 | -0,127666667 | 0,062233333 |
| BC017615 | -0,3193 | -1,535366667 | 0,068266667 | -0,278633333 | -0,4904 |
| BB246912 | 0,5739 | 0,302566667 | -0,1992 | 0,039433333 | 0,3325 |
| AF000969 | 0,294433333 | -0,820166667 | -0,559933333 | 0,6762 | -0,180933333 |
| BG066491 | -0,348866667 | -0,8219 | -0,061 | -0,627766667 | -0,177133333 |
| AF055573 | 0,368566667 | -0,197333333 | -0,168666667 | 0,0039 | -0,094566667 |
| NM_053122 | 0,1396 | -0,096466667 | -0,045133333 | -0,1158 | -0,142033333 |

### Examples

### Example 1: materials used

Dulbecco's modified Eagle's medium (DMEM), fetal calf serum (FCS), Hanks' calcium- and magnesium-free buffer, insulin and Trizol were obtained from Invitrogen (Breda, The Netherlands). Glucagon, hydrocortisone, collagenase type IV, Benzo(a)pyrene (BaP), Aflatoxin B1 (AFB1), 2-Acetylaminofluorene (2-AAF), Dimethylnitrosamine (DMN), Mitomycin C (MitC), o-Anthranilic acid (ANAC), 2-(Chloromethyl)pyridine.HCl (2-CP), 4-Nitro-o-phenylenediamine (4-NP), Quercetin (Q), 8-Hydroxyquinoline (8-HQ), Trypan blue, dimethylsulphoxide (DMSO), bovine serum albumin (BSA), 4',6-diamidino-2-phenylindole (DAPI) and Tween-20 were purchased from Sigma-Aldrich (Zwijndrecht, The Netherlands). Triton X-100, NaCl, Na₂HPO₄.2H₂O and NaH₂PO₄ were obtained from Merck (Darmstadt, Germany) and paraformaldehyde from ICN biomedicals (Auroro, Ohio). Collagen Type I Rat Tail was obtained from BD BioSciences (Bedford, MA). The RNeasy minikit was obtained from Qiagen, Westburg B.V. (Leusden, The Netherlands). The 5x MegaScript T7 Kit was obtained from Ambion (Austin, TX). The GeneChip^{®} Expression 3'-Amplification Two-Cycle cDNA Synthesis Kit and Reagents, the Hybridization, Wash and Stain Kit and the Mouse Genome 430 2.0 Arrays were purchased from Affymetrix (Santa Clara, CA).

### Example 2: Animals

Permission for performing animal studies was obtained from the Animal Ethical Committee. Adult male C57/B6 mice (Charles River), weighing 20-25 g, were obtained from Charles River GmbH, Sulzfeld, Germany. This mouse strain was chosen because it is frequently used in toxicological and pharmacological investigations, and it is a common background for transgenic mouse strains. The animals were housed in macrolon cages with sawdust bedding at 22°C and 50-60% humidity. The light cycle was 12 h light/12 h dark. Feed and tap water were available *ad libitum.*

### Example 3: Isolation of hepatocytes.

Hepatocytes were isolated from adult male C57/B6 mice by a two-step collagenase perfusion method according to Seglen and Casciano (16, 17), with modifications as described before (18). Cell viability and yield were determined by trypan blue exclusion.

### Example 4: Cell culturing and treatments.

Cells with viability >85%, were cultured in a collagen-collagen sandwich formation as described before (18, 19, 20). Prior to treatment, primary cultures of mouse hepatocytes were allowed to recover for 40-42 h at 37°C in a humidified chamber with 95%/5% air/CO₂ in serum-free culture medium supplemented with insulin 0.5U/ml), glucagon (7 ng/ml), hydrocortisone (7.5 µg/ml) and 2% penicillin/streptomycin (5000 U/ml penicillin; 5000 µm/ml streptomycin). Culture medium was refreshed every 24 h. After the recovery period, the culture medium was replaced by culture medium containing one of the selected ten compounds, or with vehicle control. Only non-cytotoxic doses were used for each compound, which were determined by the MTT assay (ca 80% viability) and are presented in Table 9. Cells were incubated for 24 or 48 h before being harvested for RNA isolation by adding Trizol reagent. Three independent replicate biological experiments with hepatocytes from different mice were conducted for each compound.

**Table 9 Solvents and dose used for several true GTX and false GTX compounds.**

| **Chemical** | **Solvent and dose (v/v %)** | **Dose** | **GTX in vitro** | **GTX in vivo** |
|---|---|---|---|---|
| | | | | |
| *True GTX compounds* | | | | |
| Benzo(a)pyrene | DMSO, 0.5 % | 30 µM | + | + |
| Aflatoxin B1 | DMSO, 0.5 % | 15 µM | + | + |
| 2-Acetylaminofluorene | DMSO, 0.5 % | 125 µM | + | + |
| Dimethylnitrosamine | | 2 mM | + | + |
| Mitomycin C | Ethanol, 0.5 % | 5 µM | + | + |
| | | | | |

| *False GTX compounds* | | | | |
|---|---|---|---|---|
| o-Anthranilic acid | DMSO, 0.5 % | 2 mM | + | - |
| 2-(Chloromethyl)pyridine.HCl | DMSO, 0.5 % | 125 µM | + | - |
| 4-Nitro-o-phenylenediamine | DMSO, 0.5 % | 2 mM | + | - |
| Quercetin | DMSO, 0.5 % | 200 µM | + | - |
| 8-Hydroxyquinoline | Ethanol, 0.5 % | 150 µM | + | - |

### Example 5: RNA isolation.

Total RNA was isolated from cultured mouse hepatocytes using Trizol and by means of the RNeasy kit according to the manufacturer's protocol. RNA concentrations were measured by means of a spectrophotometer and the quality of each RNA preparation was determined by means of a bio-analyzer (Agilent Technologies, The Netherlands). Only samples with a good quality (clear 18S and 28S peaks and RIN>6) were used for hybridization. Extracted RNA was stored at -80°C until further analysis.

### Example 6: Gene expression analysis, target preparation and hybridization.

Targets were prepared according to the Affymetrix protocol. cRNA targets were hybridized according to the manufacturer's recommended procedures on high-density oligonucleotide gene chips (Affymetrix Mouse Genome 430 2.0 GeneChip arrays). The gene chips were washed and stained using an Affymetrix fluidics station and scanned by means of an Affymetrix GeneArray scanner.

A total of eighty-two GeneChips was run. Normalization quality controls, including scaling factors, average intensities, present calls, background intensities, noise, and raw Q values, were within acceptable limits for all chips. Hybridization controls BioB, BioC, BioD, and CreX, were identified on all chips and yielded the expected increases in intensities.

### Example 7: Data analysis.

### Selection of differentially expressed probe sets.

Eighty-two datasets were obtained from this experiment. Raw data were imported into ArrayTrack (22, 23) and normalized using Robust Multi-array Average (RMA, integrated into ArrayTrack) (24).

Present-Marginal-Absent calls were used to identify and omit probe sets of poor quality (25). Subsequently, the remaining probe sets were logarithmically (base 2) transformed, corrected for vehicle control, and subjected to statistical analysis (24 h: 26100; 48 h: 26690; total: 27363). For each time point, probe sets were then selected for which expression was up- or down-regulated by at least one compound at a minimum of 1.2-fold in at least two out of three experiments with expressions altered in the same direction in all replicate and with a mean fold up- or down-regulated of 1.5 (26). The generated list with differentially expressed probe sets (log 2 ratios) was used for hierarchical clustering (HCA) and prediction analysis of microarray (PAM) (10776 probe sets at 24 h and 12180 probe sets at 48 h).

### Class prediction and functional analysis

The software tool "prediction analysis of microarray" (PAM) was used for discriminating true GTX carcinogens from false GTX carcinogens (27). PAM uses gene expression data to calculate the shrunken centroid for each class and identifies the specific genes that determine the centroid most. Based on the nearest shrunken centroid, PAM is also capable of predicting to which class an unknown sample belongs (27). Class prediction was performed after 24 h and 48 h of exposure.

For this analysis, the gene list with differentially expressed probe sets was used. For each exposure period, 3 sets of genes (classifiers) were generated by PAM, using all ten treatments, based on the smallest estimated misclassification error rate (generated by 10-fold cross-validation) and a > 80% predicted test probability. This was done by using 2 experiments as training set and the third experiment for validation. This was done for all 3 possible combinations, each time leaving out another experiment. For each time point, the classifiers that were in common between the three training sets, were set as the final classifier set for that time point

### REFERENCES

1. Dambach, et al., Toxicol Pathol, 2005. 33(1): p. 17-26.
2. Tsujimura, K., et al., Cancer Sci, 2006. 97(10): p. 1002-10.
3. Kirkland, D., et al., Mutat Res, 2005. 584(1-2): p. 1-256.
4. Chhabra, R.S., Environ Health Perspect, 1979. 33: p. 61-9.
5. Le Fevre, A.C., et al., Mutat Res, 2007. 619(1-2): p. 16-29.
6. Eun, J.W., et al., Toxicology, 2008. 249(2-3): p. 176-83.
7. Aubrecht, J., et al., Toxicol Appl Pharmacol, 1999. 154(3): p. 228-35.
8. Ellinger-Ziegelbauer, H., et al., Toxicol Lett, 2009. 186(1): p. 36-44.
9. Ellinger-Ziegelbauer, H., et al., Mutat Res, 2007.
10. Ellinger-Ziegelbauer, H., et al., Toxicol Sci, 2004. 77(1): p. 19-34.
11. Ellinger-Ziegelbauer, H., et al., Mutat Res, 2005. 575(1-2): p. 61-84.
12. Nioi, P., et al., Chem Biol Interact, 2008. 172(3): p. 206-15.
13. Waterston, R.H., et al., Nature, 2002. 420(6915): p. 520-62.
14. Koike, M., et al., J Radiat Res (Tokyo), 2008.
15. Rogakou, E.P., et al., J Biol Chem, 1998. 273(10): p. 5858-68.
16. Seglen, P.O., Methods Cell Biol, 1976. 13: p. 29-83.
17. Casciano, D.A., Drug Metab Rev, 2000. 32(1): p. 1-13.
18. Mathijs, K., et al., Drug Metab Dispos, 2009.
19. Koebe, H.G., et al., Int J Artif Organs, 1994. 17(2): p. 95-106.
20. Beken, S., et al., Methods Mol Biol, 1998. 107: p. 303-9.
21. Hamer, G., et al., Biol Reprod, 2003. 68(2): p. 628-34.
22. Tong, W., et al., Environ Health Perspect, 2003. 111(15): p. 1819-26.
23. Tong, W., et al., Mutat Res, 2004. 549(1-2): p. 241-53.
24. Irizarry, R.A., et al., Biostatistics, 2003. 4(2): p. 249-64.
25. Affymetrix, Statistical Algorithms Description Document, technical report. 2002.
26. Shi, L., et al., Nat Biotechnol, 2006. 24(9): p. 1151-61.
27. Tibshirani, R., et al., Proc Natl Acad Sci U S A, 2002. 99(10): p. 6567-72.
28. Fernandez-Capetillo, O., et al., DNA Repair (Amst), 2004. 3(8-9): p. 959-67.
29. Rogakou, E.P., et al., J Cell Biol, 1999. 146(5): p. 905-16.
30. Mladenov, E., I. Tsaneva, and B. Anachkova, J Cell Physiol, 2007. 211(2): p. 468-76.
31. Yamamoto, K., et al., Mol Med, 2008. 14(3-4): p. 167-74.
32. Zhou, C., et al., Mutat Res, 2006. 604(1-2): p. 8-18.
33. Hoogervorst, E.M., et al., DNA Repair (Amst), 2005. 4(1): p. 3-9.
34. Schrenk, D., et al., Carcinogenesis, 1994. 15(11): p. 2541-6.
35. Jenkins, G.J. and J.M. Parry, Teratog Carcinog Mutagen, 2000. 20(3): p. 107-17.
36. Moller, M.E., et al., Carcinogenesis, 1984. 5(6): p. 797-804.
37. Vu, V.T., et al., Carcinogenesis, 1985. 6(1): p. 45-52.
38. Heflich, R.H. and R.E. Neft, Mutat Res, 1994. 318(2): p. 73-114.
39. Sionov, R.V. and Y. Haupt, Oncogene, 1999. 18(45): p. 6145-57.
40. lida, M., et al., Carcinogenesis, 2005. 26(3): p. 689-99.
41. Uehara, T., et al., Toxicology, 2008. 250(1): p. 15-26.
42. van Delft, J.H., et al., Carcinogenesis, 2004. 25(7): p. 1265-76.

## Claims

1. In vitro method for predicting whether a compound is a true in vivo genotoxic compound comprising the steps of
a. providing primary mouse hepatocytes
b. providing an assay capable of determining the expression of at least one gene selected from the group consisting of the genes provided in table 1 and table 2,
c. contacting the primary mouse hepatocytes with a test compound
d. Isolating RNA from said mouse hepatocytes at two consecutive moments in time and
e. determining the expression of at least one gene selected from the group consisting of the genes provided in table 1 and table 2,
f. repeating steps a to e at least 2 times to obtain at least three measurements of expression of said at least one gene g. comparing the at least 3 measurements of expression of said at least one gene with and without the test compound at the two consecutive moment in time with data obtained from known true genotoxic compounds by means of a supervised clustering method,
h. obtaining at least 6 preliminary predictions for genotoxicity of the compound
i. predicting the true in vivo genotoxicity of the test compound according to the schedule of table 3.

2. Method according to claim 1 wherein the expression of at least two genes selected from the group consisting of the genes provided in table 1 and table 2 is determined.

3. Method according to claim 2 wherein the expression of at least two genes selected from the group consisting of the genes provided in table 1 and table 2 are determined.

4. Method according to claim 3 wherein the expression of at least three genes selected from the group consisting of the genes provided in table 1 and table 2 are determined.

5. Method according to claim 4 wherein the expression of at least ten genes selected from the group consisting of the genes provided in table 1 and table 2 are determined.

6. Method according to claims 1 to 5 wherein the two consecutive moments in time are about 24 hours and about 48 hours

7. Method according to claims 1 to 6 wherein the supervised clustering method is selected from the group consisting of PAM (Prediction Analysis of Microarray, also called Shrunken Centroids), support vector machines (SVM), k-nearest neighbours (KNN), diagonal linear discriminant analysis (DLDA), classification and regression trees (CART), probabilistic neural network (PNN) and Weighted Voting (WV).
